# EUROPEAN PATENT SPECIFICATION

(11) **EP 2 598 886 B1**
(45) Date of publication and mention of the grant of the patent: **19.08.2015**
(21) Application number: 11738203.6
(22) Date of filing: 25.07.2011
(51) Int. Cl.: G01N 33/58, G01N 33/573, G01N 33/542, C12Q 1/48

(54) **METHOD FOR THE IDENTIFICATION OF CATECHOL O-METHYLTRANSFERASE MODULATORS**
VERFAHREN ZUR IDENTIFIKATION VON CATECHOL O-METHYLTRANSFERASE-MODULATOREN
MÉTHODE D'IDENTIFICATION DE MODULATEURS DE LA CATÉCHOL O-MÉTHYLTRANSFÉRASE

(30) Priority: 27.07.2010 EP 10170957
(43) Date of publication of application: 05.06.2013
(73) Proprietor: F.Hoffmann-La Roche AG, 4070 Basel (CH)
(72) Inventor: ENDERLE, Thilo, 79618 Rheinfelden (DE); ROTH, Doris, 4123 Allschwil (CH)
(74) Representative: Brodbeck, Michel
(86) International application number: PCT/EP2011/062704
(87) International publication number: WO 2012/013614

(56) References cited:
- EP-A1- 1 674 580
- KURKELA M ET AL: "Microplate screening assay to identify inhibitors of human catechol-O-methyltransferase", ANALYTICAL BIOCHEMISTRY, ACADEMIC PRESS INC, NEW YORK, vol. 331, no. 1, 1 August 2004 (2004-08-01), pages 198-200, XP004520215, ISSN: 0003-2697, DOI: 10.1016/J.AB.2004.04.026
- HUIMIN CHEN ET AL: "Mechanisms of Quenching of Alexa Fluorophores by Natural Amino Acids", JOURNAL OF THE AMERICAN CHEMICAL SOCIETY, vol. 132, no. 21, 2 June 2010 (2010-06-02) , pages 7244-7245, XP55011331, ISSN: 0002-7863, DOI: 10.1021/ja100500k
- Anonymous: "Alexa Fluor Succinimidyl Esters", , 16 June 2009 (2009-06-16), XP002662953, Retrieved from the Internet: URL:http://probes.invitrogen.com/media/pis /mp10168.pdf [retrieved on 2011-11-07]

### SEQUENCE LISTING

<110> F. Hoffmann-La Roche AG
<120> Method for the identification of catechol O-methyltransferas modulators
<130> 26600 WO
<150> EP10170957.4
   <151> 2010-07-27
<160> 1
<170> PatentIn version 3.5
<210> 1
   <211> 271
   <212> PRT
   <213> Homo sapiens
<400> 1
   Met Pro Glu Ala Pro Pro Leu Leu Leu Ala Ala Val Leu Leu Gly Leu
   1               5                   10                  15
   Val Leu Leu Val Val Leu Leu Leu Leu Leu Arg His Trp Gly Trp Gly
               20                  25                  30
   Leu Cys Leu Ile Gly Trp Asn Glu Phe Ile Leu Gln Pro Ile His Asn
           35                  40                  45
   Leu Leu Met Gly Asp Thr Lys Glu Gln Arg Ile Leu Asn His Val Leu
       50                  55                  60
   Gln His Ala Glu Pro Gly Asn Ala Gln Ser Val Leu Glu Ala Ile Asp
   65                  70                  75                  80
   Thr Tyr Cys Glu Gln Lys Glu Trp Ala Met Asn Val Gly Asp Lys Lys
                   85                  90                  95
   Gly Lys Ile Val Asp Ala Val Ile Gln Glu His Gln Pro Ser Val Leu
               100                 105                 110
   Leu Glu Leu Gly Ala Tyr Cys Gly Tyr Ser Ala Val Arg Met Ala Arg
           115                 120                 125
   Leu Leu Ser Pro Gly Ala Arg Leu Ile Thr Ile Glu Ile Asn Pro Asp
       130                 135                 140
   Cys Ala Ala Ile Thr Gln Arg Met Val Asp Phe Ala Gly Val Lys Asp
   145                 150                 155                 160
   Lys Val Thr Leu Val Val Gly Ala Ser Gln Asp Ile Ile Pro Gln Leu
                   165                 170                 175
   Lys Lys Lys Tyr Asp Val Asp Thr Leu Asp Met Val Phe Leu Asp His
               180                 185                 190
   Trp Lys Asp Arg Tyr Leu Pro Asp Thr Leu Leu Leu Glu Glu Cys Gly
           195                 200                 205
   Leu Leu Arg Lys Gly Thr Val Leu Leu Ala Asp Asn Val Ile Cys Pro
       210                 215                 220
   Gly Ala Pro Asp Phe Leu Ala His Val Arg Gly Ser Ser Cys Phe Glu
   225                 230                 235                 240
   Cys Thr His Tyr Gln Ser Phe Leu Glu Tyr Arg Glu Val Val Asp Gly
                   245                 250                 255
   Leu Glu Lys Ala Ile Tyr Lys Gly Pro Gly Ser Glu Ala Gly Pro
               260                 265                 270

## Description

The present invention relates to a method for the identification of modulators of catechol O-methyltransferase enzyme activity.

Catechol O-methyltransferase (COMT) catalyzes the O-methlyation of substrates that have a catechol moiety. The methyl donor in the methylation by COMT is S-adenosylmethionine (SAM). COMT plays an important role for the catabolism of endogenous catecholamine neurotransmitters, catecholestrogens and xenobiotic molecules. Inhibition of COMT is an important approach for developing new therapeutic treatments in Parkinson's disease.

W.F. Herblin (Analytical Biochemistry 51, 19-22, 1973) describes a colorimetric assay for COMT activity. The assay uses nitrocatechol as methyl acceptor for COMT. Nitrocatechol exists as a yellow solution in water at acid pH with an absorption maximum at 350 nm. At slightly alkaline pH ionization of the para hydroxyl turns the solution orange (λmax = 430 nm). In stronger alkali ionization of the meta hydroxyl leads to a cherry red solution (λmax = 520 nm). The assay is based on the observations that nitrocatechol is methylated by COMT and that methylated nitrocatechol no longer exhibits the cherry red color resulting from the second ionization. In this assay the substrate (nitrocatechol) and SAM have to be in the µM concentration range which is at or above Kₘ what is limiting the sensitivity.

G. Zürcher and M. Da Prada (Journal of Neurochemistry, Vol. 38, No. 1, 1982) describe a Single step radiochemical assay for COMT activity. In this assay, catechol is converted to tritiated guajacol, a compound of very low polarity, by incubating COMT with [³H]methyl SAM, Mg²⁺ and adenosine deaminase. Guajacol is extracted using a low polarity medium e.g. Toluene, and counted in a scintillation counter.

The above described assays are not suitable for automated screening large numbers of compounds for their COMT modulator activity due to limited sensitivity (colorimetric assay) or due to the assay setup (extraction step in the radiochemical assay).

Therefore, there is a need for a sensitive, homogeneous assay method suitable for screening large numbers of compounds for their COMT modulating activity.

In a first object the present invention provides a method for the identification of a modulator of the activity of a catechol O - methyl transferase enzyme (COMT) comprising the steps of:
a) providing 4-nitrocatechol covalently linked to Alexa Fluor 488 having formula I,
b) contacting the molecule of step a) with a catechol O - methyl transferase enzyme (COMT), a S-adenosylmethionine (SAM) and a candidate compound and
c) measuring the fluorescence readout of the mixture of step b), wherein an altered fluorescence readout in presence of the candidate compound compared to a blank is indicative for a modulator of a catechol O-methyl transferase enzyme (COMT),

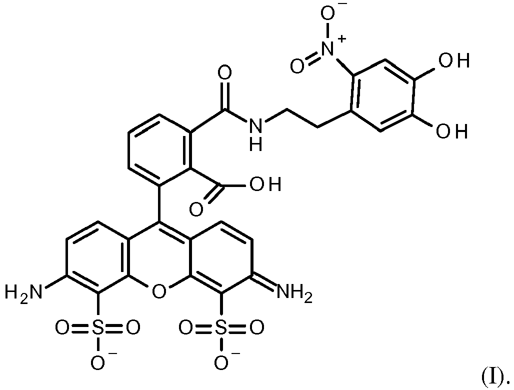

In a preferred embodiment, the method is a method for the identification of a COMT inhibitor, wherein a decreased fluorescence readout in step c) compared to a blank is indicative for a COMT inhibitor.

In a further preferred embodiment the fluorescence readout in step c) is a kinetic readout.

In a further preferred embodiment the COMT is human COMT.

In a further preferred embodiment the method is a High Throughput screening method.

In a further preferred embodiment the method is performed in a microtiter plate.

In a further preferred embodiment the final concentration of COMT is about 25nM.

In a further preferred embodiment the final concentration of the COMT substrate is about 200 nM.

In a further preferred embodiment the final concentration of SAM is about 500nM.

In a second object, the present invention provides a method for the identification of a substrate of a catechol O- methyl transferase enzyme (COMT) comprising the steps of:
a) providing 4-nitrocatechol covalently linked to Alexa Fluor 488 having formula I and S-adenosylmethionine (SAM),
b) contacting the mixture of step a) with different concentrations of a candidate compound,
c) contacting the mixtures of step b) with a catechol O- methyl transferase enzyme (COMT) and
d) measuring the kinetic fluorescence readout of the mixtures of step c), wherein a decreasing fluorescence readout plateau as a function of an increasing concentration of the candidate compound is indicative for a substrate of a catechol O-methyl transferase enzyme (COMT),

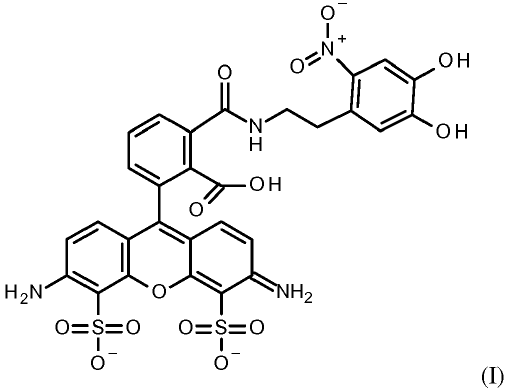

### Short description of the figures

Fig. 1 shows the chemical structure of Alexa Fluor^{®} 488 covalently coupled to 4-Nitrocatechol;
Fig. 2 shows a Stern Volmer Plot for nitrocatechol (blue), 2-methoxy-5-nitrophenol (red) and 1,2-dimethoxy-4-nitrobenzene (green); 20 nM free Alexa Fluor 488 was mixed with high - up to 25 mM - concentrations of nitrocatechol, 2-methoxy-5-nitrophenol and 1,2-dimethoxy-4-nitrobenzene, respectively. Only for nitrocatechol a change of the fluorescence intensity (I₀/I) of Alexa Fluor^{®} 488 is observed, the methylated products do not influence the fluorescence intensity of Alexa Fluor^{®} 488.
Fig. 3 shows the enzyme kinetics of the COMT catalyzed methylation of Alexa Fluor 488-Nitrocatechol in the fluorescence assay of the present invention;
Fig. 4a shows a kinetic measurement of the change of the fluorescence intensity in presence of various concentrations of the COMT inhibitor Tolcapone;
Fig. 4b shows the dose response curve for Tolcapone calculated from the slopes of the kinetic measurement of Fig. 3a;
Fig. 5 shows the fluorescent assay in presence of dopamine, a natural substrate of COMT. Decreasing plateaus were reached with increasing dopamine concentrations. As dopamine is a substrate it is methylated as well as the Alexa Fluor^{®} 488-Nitrocatechol substrate. The availability of SAM is limited (500 nM) so that at high dopamine concentrations the Alexa Fluor^{®} 488-Nitrocatechol substrate can no longer get fully methylated.

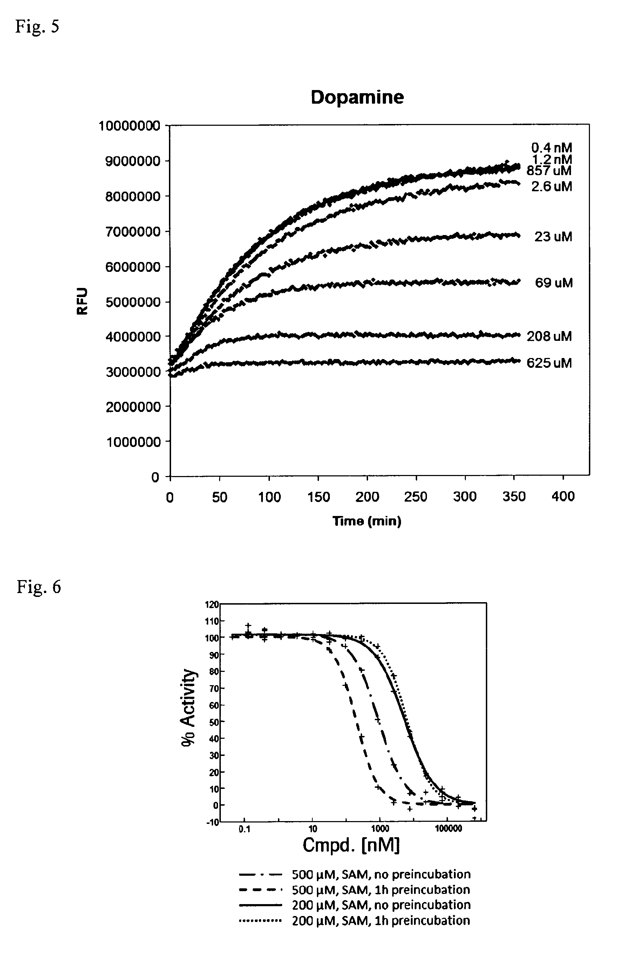

Fig. 6 shows the dose response curves for a substrate with low (500 nM) and high (200 µM) SAM concentrations and for each SAM concentration with and without one hour pre-incubation of compound and SAM before adding the Alexa Fluor^{®} 488-Nitrocatechol substrate. The pre-incubation with low SAM shifts the dose response curve to a lower IC₅₀ because the compound uses up SAM. At high SAM concentrations there is no difference between with and without pre-incubation because SAM is not limiting and the dose response curve is shifted to higher IC₅₀ compared to low SAM because the compound is used up.
Fig. 7 shows dose response curves with low (500 nM) and high (200 µM) SAM concentrations again with and without one hour pre-incubation of the compound with SAM of a SAM competitive compound. For a SAM competitive compound with and without pre-incubation does not affect the IC₅₀ for each SAM concentration but with high SAM concentration the IC₅₀ is shifted to larger values.

### Detailed description of the invention

The assay of the present invention is based on the findings that a fluorescent dye covalently coupled to a COMT substrate e.g. Alexa Fluor^{®} 488 covalently linked to nitrocatechol, shows a decreased fluorescence due to intramolecular quenching and that methylation of the COMT substrate in the COMT substrate-fluorescent dye complex by COMT abolishes quenching of the fluorescence i.e. methylation of the COMT substrate in the COMT substrate - fluorescent dye complex leads to an increased fluorescence compared to the non methylated complex.

The term "COMT" is used herein to refer to native sequence COMT from any animal, e.g. mammalian, species, including humans, and COMT variants (which are further defined below). The COMT polypeptides may be isolated from a variety of sources, including human tissue types or prepared by recombinant and/or synthetic methods.

Natural or recombinantly produced COMT can be used in this assay. A "recombinant protein" is a protein isolated, purified, or identified by virtue of expression in a heterologous cell, said cell having been transduced or transfected, either transiently or stably, with a recombinant expression vector engineered to drive expression of the protein in the host cell. Recombinant COMT can be produced in procaryotic cells e.g. *E.coli,* in yeast e.g. *S.pombe* or in eukaryotic cells e.g. HEK 293, Sf9 insect cells. Preferably, Sf9 insect cells are used for high expression of recombinant COMT. The COMT used in the assay may be purified. The term "purified" as used herein refers to polypeptides, that are removed from their natural environment or from the source of recombinant production, isolated or separated, and are at least 60% and more preferably at least 80% free from other components, e.g. membranes and microsomes, with which they are naturally associated.

"Native sequence COMT" refers to a polypeptide having the same amino acid sequence as a COMT polypeptide occurring in nature regardless of its mode of preparation. A native sequence COMT may be isolated from nature, or prepared by recombinant and/or synthetic methods. The term "native sequence COMT" specifically encompasses naturally occurring truncated or secreted forms, naturally occurring variant forms (e.g. alternatively spliced forms), and naturally occurring allelic variants of COMT. The identifier of the human COMT polypeptide in the NCBI database is AAA68927 (Seq. Id. No. 1).

The term "COMT variant" refers to amino acid sequence variants of a native sequence COMT, containing one or more amino acid substitution and/or deletion and/or insertion in the native sequence. The amino acid sequence variants generally have at least about 75%, preferably at least about 80%, more preferably at least about 85%, even more preferably at least about 90%, most preferably at least about 95% sequence identity with the amino acid sequence of a native sequence COMT.

The term "compound" is used herein in the context of a "test compound" or a "drug candidate compound" described in connection with the assays of the present invention. As such, these compounds comprise organic or inorganic compounds, derived synthetically or from natural sources. The compounds include inorganic or organic compounds such as polynucleotides, lipids or hormone analogs that are characterized by relatively low molecular weights. Other biopolymeric organic test compounds include peptides comprising from about 2 to about 40 amino acids and larger polypeptides comprising from about 40 to about 500 amino acids, such as antibodies or antibody conjugates.

The term "kinetic readout" refers to the difference of the fluorescent signal measured at two certain time points in the linear part of an enzymatic reaction. One measurement is done at the beginning of the enzymatic reaction (start-point) and after an incubation time a second readout is performed (end-point). The final signal is then calculated in rfu/min as (rfu(end-point) - rfu(start-point)) / incubation time. (rfu: relative fluorescence units)

The method of the present invention can by employed to identify compounds which inhibit the enzyme Catechol O-methyltransferase (COMT). Thus, COMT inhibitors identified by the method of the present invention can be used in methods for the treatment, prevention, or control of illnesses in which a deactivation of extraneuronal catecholamines by COMT plays a role, for example, in the prevention or control of depressions. In this case the compounds of the invention can be used as individual compounds or in combination with other therapeutically active substances which favorably influence the course of the illness. The compounds of the invention can also be used as co-medications with other therapeutically active substances.

The method of the present invention can be used to determine COMT activity in tissue samples of animals which have been treated with a test compound. For example, the assay is suitable to determine COMT activity in brain and liver tissue samples from animals e.g. mice and rats, which have been treated with a test compound (COMT modulator).

### Experimental Part

### Synthesis of 4-Nitrocatechol - Alexa Fluor^{®} 488

A 10 mM solution of Aminoethyl-nitro-brenzcatechin [1] in DMSO containing 1% Triethylamine was mixed with a 10 mM solution of Alexa Fluor@ 488 carboxylic acid succinimidyl ester [2] (Invitrogen Corporation, 5791 Van Allen Way, Carlsbad, California 92008) in DMSO with 1% Trietylamine in a 1:1 soichiometric ratio. The reaction mixture was gently mixed over night at room temperature and purified on a Äkta Explorer 100 Reversed-Phase-HPLC. The product was lyophilized and resuspended in DMSO.

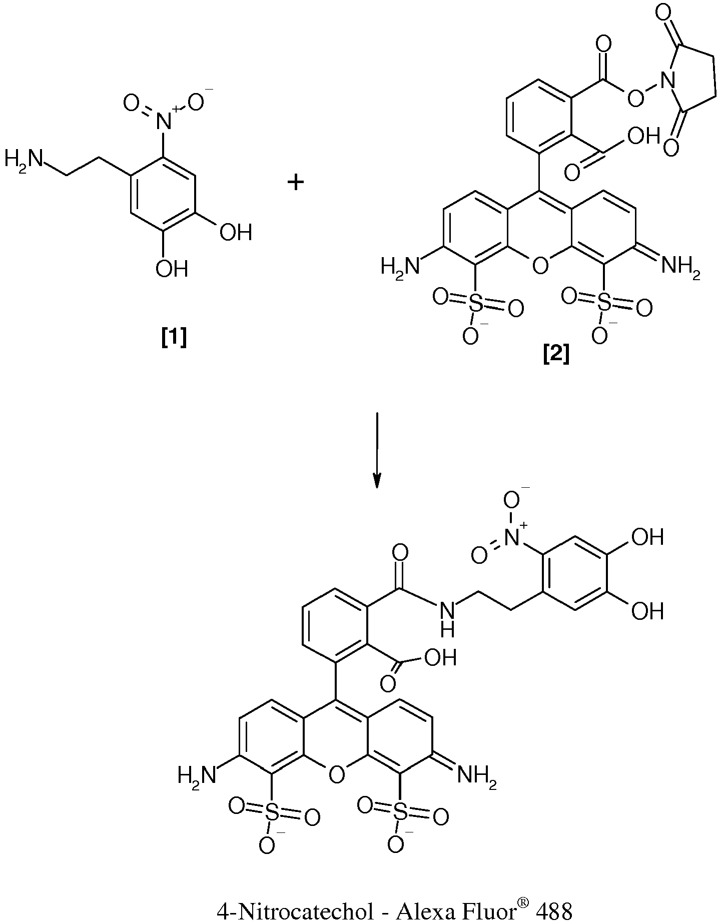

### Fluorescence assay protocol

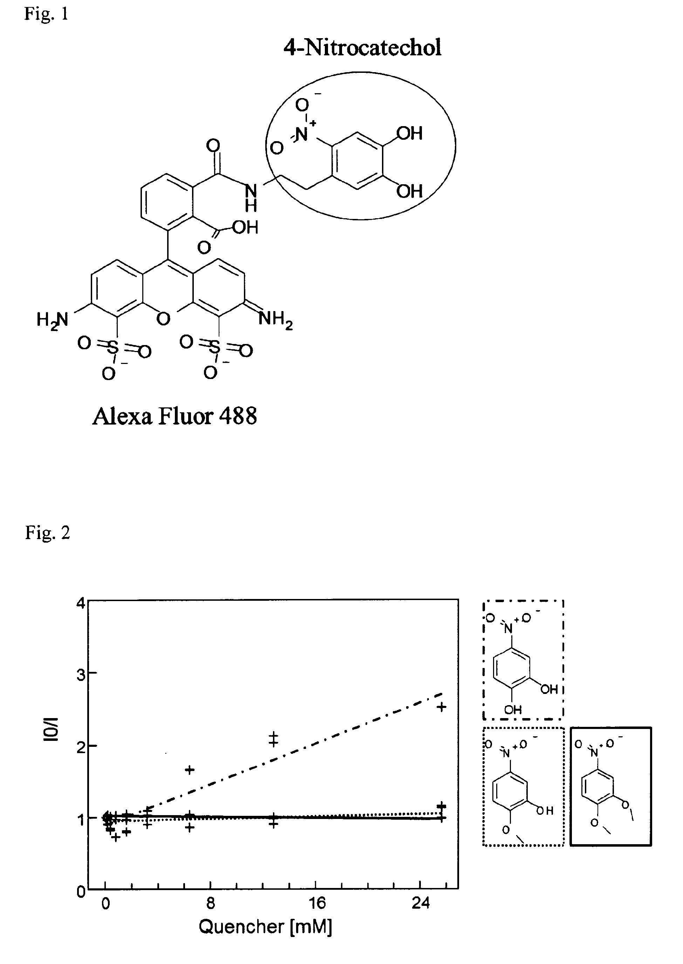

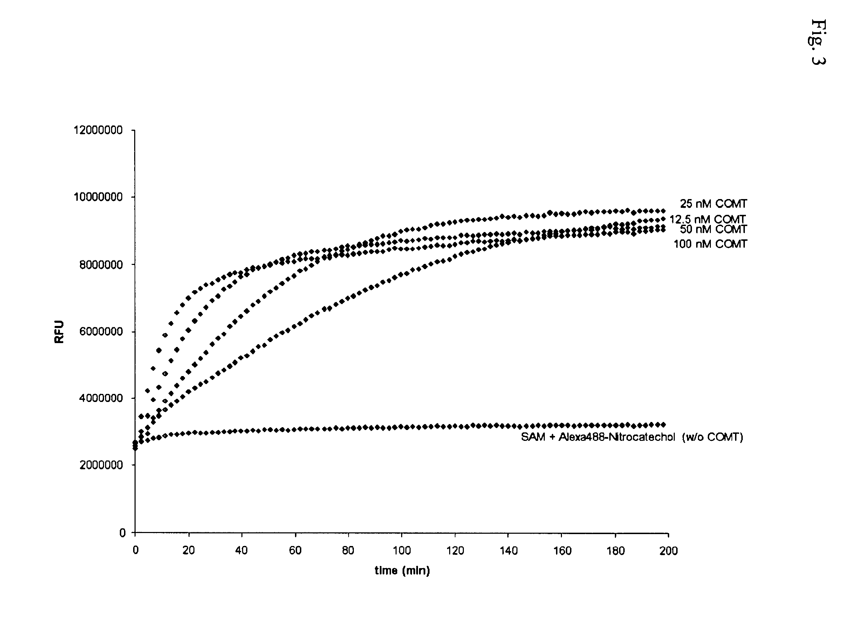

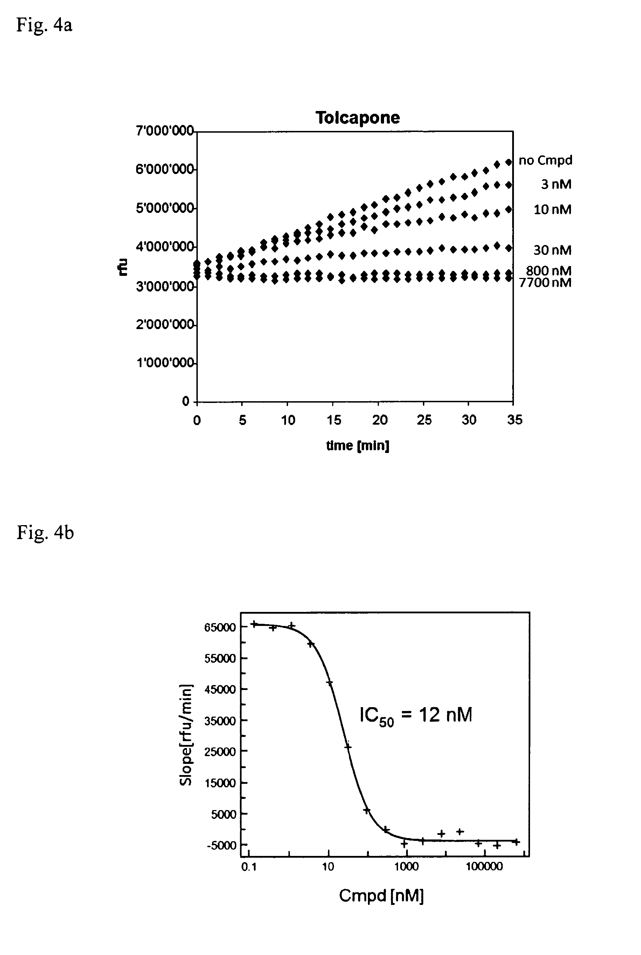

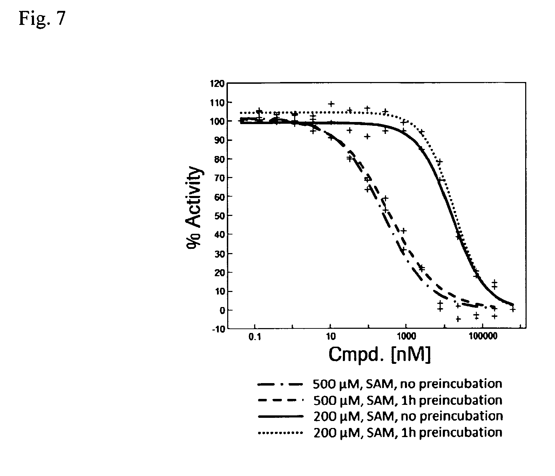

The following assay protocol, reagents and materials were used in the examples of the present invention. The results of the examples are described in the figures 1 - 7.

### Microtiterplates:

384-well microtiter plate, Corning black with flat clear bottom, non binding surface, polystyrene (ref. 3655)

### Reagent and buffer stock solutions:

- Buffer stock solutions:
   - M Phosphate buffer pH 7.6 (Na₂HPO₄ Fluka 71644, NaH₂PO₄ Merck 6346.0500) stored at 4°C
   - 580 mM MgCl₂ (Merck 1.0833.0250), stored at RT
   - 1 M CaCl₂ stored at 4°C
   - 65 mM DTT (Sigma D-0632), stored at -20°C
- Recombinant human COMT: prepared in house, stored at -80°C
- 4-Nitrocatechol-Alexa Fluor 488: prepared in house, 1.3 mM in DMSO, stored at RT in the dark
- S-Adenosyl-methionine: 10 mM in H₂O (Sigma-Aldirch A2804), stored at -20°C

### Reagent and buffer solutions:

- Assaybuffer (end concentrations):
   - 40 mM Phosphate buffer pH 7.6
   - 2.88 mM MgCl₂
   - 0.9 mM DTT
   - 0.25 mM CaCl₂
- Compound dilutions: dilutions in 100% DMSO (Sigma 41640), 6.25% DMSO final assay concentration
- Rec. human COMT: 80 nM in assay buffer, 25 nM final assay concentration
- 4-Nitrocatechol- Alexa Fluor^{®} 488: 320 nM in assay buffer, 200 nM final assay concentration
- S-Adenosyl-methionine: 800 nM in assay buffer, 500 nM final assay cencentraion

### Assay Method:

10 µl hCOMT (human COMT)
2 µl test compound
1 min on shaker
20 µl Substrate-SAM-Mix
5 min on shaker

Readout: kinetic measurement on plate:: vision TM reader (exc. 475(40) nm, em. 535(45) nm, intensity 7.5 %, exposure time Is.

## Claims

1. A method for the identification of a modulator of the activity of a catechol O - methyl transferase enzyme (COMT) comprising the steps of:
a) providing 4-nitrocatechol covalently linked to Alexa Fluor 488 having formula I,
b) contacting the molecule of step a) with a catechol O - methyl transferase enzyme (COMT), a S-adenosylmethionine (SAM) and a candidate compound and
c) measuring the fluorescence readout of the mixture of step b), wherein an altered fluorescence readout in presence of the candidate compound compared to a blank is indicative for a modulator of a catechol O-methyl transferase enzyme (COMT),

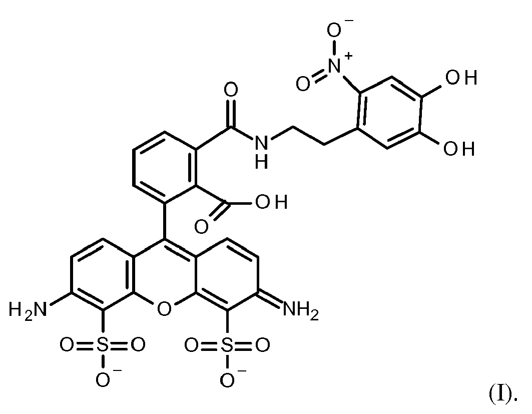

2. The method of claim 1, wherein the method is a method for the identification of a COMT inhibitor and a decreased fluorescence readout in step c) compared to a blank is indicative for a COMT inhibitor.

3. The method of anyone of the preceding claims, wherein the fluorescence readout in step c) is a kinetic readout.

4. The method of anyone of the preceding claims, wherein the COMT is human COMT.

5. The method of anyone of the preceding claims, wherein the method is a High Throughput screening method.

6. The method of anyone of the preceding claims, wherein the method is performed in a microtiter plate.

7. The method of anyone of the preceding claims, wherein the final concentration of COMT is about 25nM.

8. The method of anyone of the preceding claims, wherein the final concentration of the COMT substrate is about 200 nM.

9. The method of anyone of the preceding claims, wherein the final concentration of SAM is about 500nM.

10. A method for the identification of a substrate of a catechol O- methyl transferase enzyme (COMT) comprising the steps of:
a) providing 4-nitrocatechol covalently linked to Alexa Fluor 488 having formula I and S-adenosylmethionine (SAM),
b) contacting the mixture of step a) with different concentrations of a candidate compound,
c) contacting the mixtures of step b) with a catechol-O-methyl transferase enzyme (COMT) and
d) measuring the kinetic fluorescence readout of the mixtures of step c), wherein a decreasing fluorescence readout plateau as a function of an increasing concentration of the candidate compound is indicative for a substrate of a catechol-O-methyl transferase enzyme (COMT),

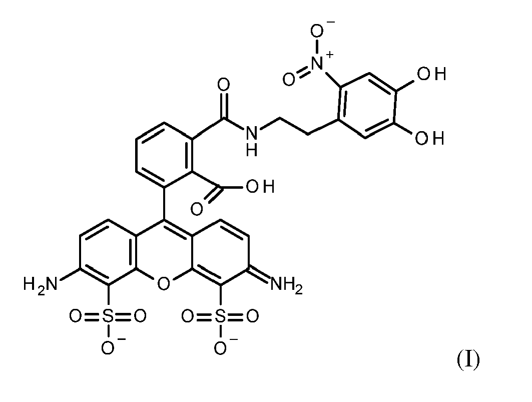

## Patentansprüche

1. Verfahren zum Identifizieren eines Modulators der Aktivität eines Catechol-O-Methyltransferase-Enzyms (COMT), umfassend die Schritte:
a) Bereitstellen von 4-Nitrocatechol, das kovalent an Alexa Fluor 488 gebunden ist, das die Formel I hat,
b) Inkontaktbringen des Moleküls aus Schritt a) mit einem Catechol-O-Methyltransferase-Enzym (COMT), einem S-Adenosylmethionin (SAM) und einer Kandidatenverbindung, und
c) Messen des Fluoreszenz-Readouts des Gemischs aus Schritt b), wobei ein verändertes Fluoreszenz-Readout bei Vorliegen der Kandidatenverbindung verglichen mit einer Leerprobe einen Modulator eines Catechol-O-Methyltransferase-Enzyms (COMT) anzeigt,

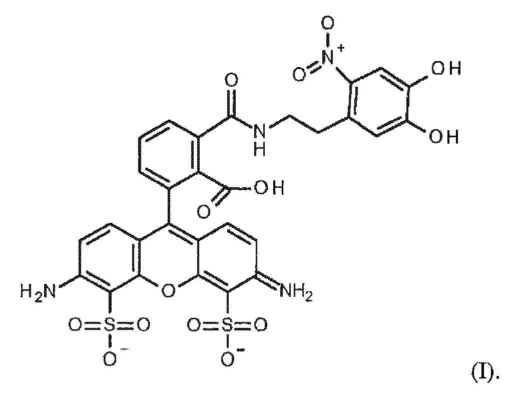

2. Verfahren nach Anspruch 1, wobei das Verfahren ein Verfahren zum Identifizieren eines COMT-Inhibitors ist und wobei ein verringertes Fluoreszenz-Readout in Schritt c) verglichen mit einer Leerprobe einen COMT-Inhibitor anzeigt.

3. Verfahren nach einem der vorangehenden Ansprüche, wobei das Fluoreszenz-Readout im Schritt c) ein kinetisches Readout ist.

4. Verfahren nach einem der vorangehenden Ansprüche, wobei das COMT humanes COMT ist.

5. Verfahren nach einem der vorangehenden Ansprüche, wobei das Verfahren ein Hochdurchsatz-Screening-Verfahren ist.

6. Verfahren nach einem der vorangehenden Ansprüche, wobei das Verfahren in einer Mikrotiterplatte durchgeführt wird.

7. Verfahren nach einem der vorangehenden Ansprüche, wobei die Endkonzentration des COMT etwa 25 nM ist.

8. Verfahren nach einem der vorangehenden Ansprüche, wobei die Endkonzentration des COMT-Substrats etwa 200 nM ist.

9. Verfahren nach einem der vorangehenden Ansprüche, wobei die Endkonzentration von SAM etwa 500 nM ist.

10. Verfahren zum Identifizieren eines Substrats eines Catechol-O-Methyltransferase-Enzyms (COMT), umfassend die Schritte:
a) Bereitstellen von 4-Nitrocatechol, das kovalent an Alexa Fluor 488 gebunden ist, das die Formel I hat und S-Adenosylmethionin (SAM),
b) Inkontaktbringen des Gemischs aus Schritt a) mit verschiedenen Konzentrationen einer Kandidatenverbindung,
c) Inkontaktbringen der Gemische aus Schritt b) mit einem Catechol-O-Methyltransferase-Enzym (COMT), und
d) Messen des kinetischen Fluoreszenz-Readouts der Gemische aus Schritt c), wobei ein sich verringerndes Fluoreszenz-Readout-Plateau als Funktion einer sich erhöhenden Konzentration der Kandidatenverbindung ein Substrat eines Catechol-O-Methyltransferase-Enzyms (COMT) anzeigt,

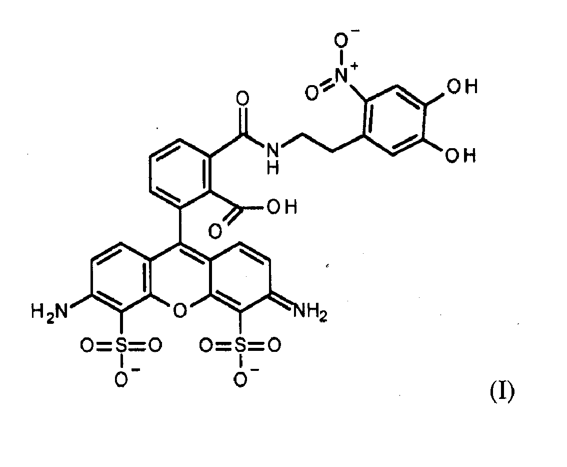

## Revendications

1. Procédé pour l'identification d'un modulateur de l'activité d'une enzyme catéchol-O-méthyltransférase (COMT), comprenant les étapes de :
a) fourniture de 4-nitrocatéchol lié par covalence à de l'Alexa Fluor 488 répondant à la formule I,
b) mise en contact de la molécule de l'étape a) avec une enzyme catéchol-O-méthyltransférase (COMT), une S-adénosylméthionine (SAM) et un composé candidat, et
c) mesure de la valeur de lecture de fluorescence du mélange de l'étape b), une valeur de lecture de fluorescence modifiée en présence du composé candidat comparativement à un témoin à blanc étant indicative d'un modulateur d'une enzyme catéchol-0-méthyltransférase (COMT),

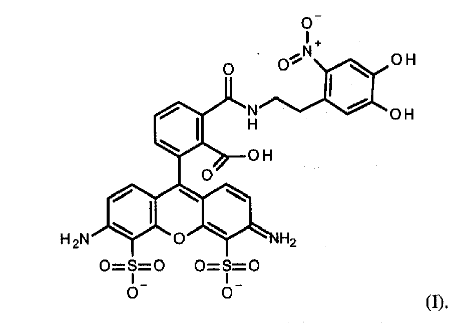

2. Procédé suivant la revendication 1, ledit procédé étant un procédé pour l'identification d'un inhibiteur de COMT et une valeur de lecture de fluorescence réduite dans l'étape c) comparativement à un témoin à blanc étant indicative d'un inhibiteur de COMT.

3. Procédé suivant l'une quelconque des revendications précédentes, dans lequel la valeur de lecture de fluorescence dans l'étape c) est une valeur de lecture cinétique.

4. Procédé suivant l'une quelconque des revendications précédentes, dans lequel la COMT est la COMT humaine.

5. Procédé suivant l'une quelconque des revendications précédentes, le procédé étant un procédé de dépistage à haut débit.

6. Procédé suivant l'une quelconque des revendications précédentes, le procédé étant mis en oeuvre dans une plaque de microtitrage.

7. Procédé suivant l'une quelconque des revendications précédentes, dans lequel la concentration finale de COMT est d'environ 25 nM.

8. Procédé suivant l'une quelconque des revendications précédentes, dans lequel la concentration finale du substrat de COMT est d'environ 200 nM.

9. Procédé suivant l'une quelconque des revendications précédentes, dans lequel la concentration finale de SAM est d'environ 500 nM.

10. Procédé pour l'identification d'un substrat d'une enzyme catéchol-O-méthyltransférase (COMT), comprenant les étapes de :
a) fourniture de 4-nitrocatéchol lié par covalence à de l'Alexa Fluor 488 répondant à la formule I, et de S-adénosylméthionine (SAM),
b) mise en contact du mélange de l'étape a) avec différentes concentrations d'un composé candidat,
c) mise en contact des mélanges de l'étape b) avec une enzyme catéchol-O-méthyltransférase (COMT), et
d) mesure de la valeur de lecture de fluorescence cinétique des mélanges de l'étape c), un plateau de valeurs de lecture de fluorescence réduit en fonction d'une concentration croissante du composé candidat étant indicatif d'un substrat d'une enzyme catéchol-O-méthyltransférase (COMT),
